(19)

**Europäisches Patentamt**

**European Patent Office**

**Office européen des brevets**

(11) **EP 4 691 226 A1**

(12) **EUROPEAN PATENT APPLICATION**
published in accordance with Art. 153(4) EPC

(43) Date of publication:
**11.02.2026 Bulletin 2026/07**

(21) Application number: **24777342.7**

(22) Date of filing: **26.03.2024**

(51) International Patent Classification (IPC):
**A01H 4/00** (2006.01)     **A01H 6/46** (2018.01)
**A01G 22/55** (2018.01)     **C12M 3/02** (2006.01)

(52) Cooperative Patent Classification (CPC):
**A01G 22/55; A01H 4/00; A01H 6/46; C12M 3/02**

(86) International application number:
**PCT/BR2024/050119**

(87) International publication number:
**WO 2024/197370 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 BR 102023006175**

(71) Applicant: **CTC - Centro de Tecnologia Canavieira S.A.**
**13400-970 Piracicaba - SP (BR)**

(72) Inventors:
• **FERREIRA, Willian Jonas**
**13400-970 Piracicaba, SP (BR)**
• **FINI, Ricardo, Evaristo**
**13400-970 Piracicaba, SP (BR)**
• **CALZADO, Felipe**
**13400-970 Piracicaba, SP (BR)**

(74) Representative: **Elzaburu S.L.P.**
**Paseo de la Castellana 259C**
**Torre de Cristal, planta 28**
**28046 Madrid (ES)**

(54) **CELL AGGREGATE CULTIVATION PROCESS, CELL AGGREGATES, PLANT CELL MICROPROPAGATION METHOD AND SOMATIC EMBRYO MATURATION METHOD**

(57)     The present invention relates to the field of biotechnology. More precisely, a process of growing cell aggregates in a system of containers rotating around their own horizontal axes is described. The invention also describes the cell aggregates used in the cultivation process and a method of micropropagating plant cells that comprises inoculating the plant cells in said containers with rotation around their own horizontal axes.

**Figure 1**

**Description**

Technical Field

The present invention relates to the field of biotechnology. More precisely, a process for cultivating cell aggregates in a system of containers with controlled rotation around their own horizontal axes is described. In addition, the cell aggregates cultivated from this process are described, as well as a method of micropropagating plant cells that involves cultivating cell aggregates from plant explants in said containers with controlled rotation around their horizontal axes.

State of the Art Description

Cell culture techniques have been widely used due to the growing need to establish cell lines in *ex vivo* environments. Such cell cultures are used not only for the production of compounds of interest by the cells, but also for functional and pharmacological studies, for the production of tissues and organs, for genetic transformation, mutagenesis, editing and for plant propagation.

Culturing animal cells, for example, has become indispensable in the field of life sciences, as it makes it possible to study cell growth and differentiation, identify the growth factors involved in these stages and increase understanding of the mechanisms underlying the normal functions of various types of cells.

With regard to plant cell cultures, their importance in the production of compounds of interest stands out, with the possibility of decoupling such production from geographical and environmental conditions, eliminating dependence on conventional cultivation systems and wild plants (Espinosa-Leal et al., 2018, Planta 248, 1-18). Furthermore, plant cell cultures enable the propagation of plants for commercial purposes, plant genetic manipulation, for example, through genetic transformation methods, mutagenesis and gene editing, in addition to being important tools for conventional plant breeding and seedling sanitation, enabling healthier plantings.

*In vitro* plant cell culture systems are generally based on growing plant explants in suitable culture media from which the tissues multiply and regenerate into mature plants.

For example, the propagation and regeneration of plants by tissue culture techniques is widely known in the state of the art and numerous publications suggest micropropagation techniques and methods for a wide variety of plant species, with successful reports for *in vitro* micropropagation via organogenesis or somatic embryogenesis (direct or indirect) of certain plant species. The production of sugarcane seedlings, for example, can be achieved by micropropagation, which is widely described in the state of the art (Franklin et al., 2006, Plant Growth Regul 50: 111; Naz et al., 2008, Sarhad J. Agric. 24 (4): 593). Sugarcane propagules for commercial purposes are produced via direct organogenesis (Burner & Grisham, 1995, Crop Sci. 35:875) or indirect somatic embryogenesis (Liu, 1993, J. Plant Physiol. 141:714; Brisibe et al., 1994, New Phytol. 126: 301; Blanco et al., 1997, Plant Cell Tiss Organ Cul 51: 153) using apical meristem and callus produced from immature leaves (palm hearts), respectively, as explants.

Micropropagation through organogenesis leads to the formation of an organ such as an aerial part or root, which can be isolated to induce the development of roots or shoots to produce the whole plant. The direct shoot multiplication systems (direct organogenesis) currently in use do not have the necessary scale to make a significant impact on commercial planting and are too costly to compete with conventional multiplication systems. On the other hand, *in vitro* micropropagation methods using somatic embryogenesis are based on the production of embryos *in vitro* from small pieces of plant tissue or individual cells, and are widely described in the state of the art (Vasil, 1987, J. Plant Physiol. 128: 192; Manickavasagan & Ganapathi, 1998, Ind. J. Exp. Bio. 36: 832). Somatic embryos are bipolar structures that contain both stem and root meristems and have no vascular connection with the parental tissue. They can be formed from callus (indirect somatic embryogenesis) or from suspension cell culture (direct somatic embryogenesis), leading to the formation of mature plants. For somatic embryogenesis to be practical for large-scale production, numerous problems must be overcome which, up to the time of the present invention, represent obstacles to plant production.

Ali et al. (2007) (Pak. J. Bot. 39(6): 1961) reported that the induction of direct somatic embryogenesis in sugarcane was dependent on the type of explant, hormone supplementation and light and dark conditions. Among the different explants used, the leaf explant showed the highest frequency of embryo induction. It was observed that the direct somatic embryos appeared at the edge of the leaf cut incubated in 16 hours in the light and 8 hours in the dark.

Rui & Seppo (2004) (Agri and Food Sci. 13(4): 363) reported in rye that the factors influencing somatic embryogenesis of immature embryos are: genotype, culture medium, sucrose, polymerizing agent and auxin.

Himanshu et al. (2000) (Ind. J. of Agr. Sci 70 (3): 181), Chengalrayan & Meagher (2001) (In Vitro Cell & Devel. Biol. Plant 37(4): 434) used leaf explants for somatic embryogenesis in sugarcane.

Lakshmanan et al. (2006) (Plant Cell Rep 25(10): 1007) used cross-sections of young leaves close to the meristem to induce regeneration. According to the authors, the polarity of the tissue and the orientation of the explant in culture, the size and stage of development of the explant, and the concentration of auxin were determining factors for the organogenic potential of the leaf tissue in culture. The production of adventitious shoots and somatic embryogenesis occurred on the proximal surface of the explant cut. Cross-sections (1 to 2 mm) obtained from curled young leaves and the orientation of the explants with their distal face in contact with the medium (directly in contact with the medium) were identified as critical for maximum regeneration. Shoot regeneration was observed in 3 weeks on MS medium supplemented with NAA and BAP, while somatic embryogenesis or both organogenic adventitious shoots and somatic embryogenesis occurred on medium with NAA and CPA. Shoots between 20 and 30 mm long were grown in root culture medium for 6 weeks of incubation. Around 90% of the 30 to 40 mm long shoots were transferred to trays with substrate and kept in a greenhouse with high humidity and shading. This technology aims to propagate new cultivars and transgenic sugarcane plants. This sugarcane plant production process lasts 4 months, including the micropropagation stage in the laboratory and the seedling acclimation stage in a greenhouse, and requires a lot of labor to individualize the seedlings produced in vitro and transplant them into trays with substrate. Currently, this seedling production process does not have the necessary scale to make a significant impact on commercial planting.

Although there is the capacity for plant parts and cells to regenerate complete plants called totipotents, specialized studies are required to adapt the conditions that allow regeneration. Some widely applied factors controlling the growth and differentiation of each culture are determined. The establishment of interactions between different groups of phytoregulators in combinations or in isolation is responsible for the interrelationships that exist between cells, tissues and organs. There seems to be a consensus that success in inducing differentiation depends on the type of explant, the physiological condition of the explant and the chemical and physical environment of the explant during culture. Because of this, the science of tissue culture focuses on perfecting the physiological conditions of the plant of origin, the type of explant, the culture conditions and the phyto-regulators used to initiate tissue culture. This confirms the fact that the development of a new process for plant multiplication by tissue culture is not obvious.

In this regard, it is clear that the cultivation of callus in the process of indirect somatic embryogenesis is a potential system for the large-scale production of plants and plant material for genetic manipulation, but it presents important technical problems that must be fully overcome in order to achieve high productivity.

For example, prolonged maintenance of callus *in vitro* and repeated subcultivation to increase the scale of the process can lead to genetic instability and increase the chance of somaclonal variation (LAKSHMANAN et. al, 2006). In addition, the cultivation of callus in liquid media is still carried out using non-specific bioreactors, usually in Erlenmeyers with limited working volumes and requiring large numbers of personnel to handle, making the process more expensive. Even if industrial scale can be achieved through numerous cultivations and subcultures, the embryos derived from callus produced by current methods do not have the necessary quality, largely because the aggregated cell mass is not all at the same stage of development, as the cells of the cell aggregate do not have sufficient homogeneity of nutrients and oxygen to be all at the same level of development.

The use of bioreactors to cultivate pro-embryogenic masses and produce somatic embryos has been successful in some dicotyledons (Holst et al., 1996 US 5,587,312; Keiichirou and Noburu, 1995 US 5,413,929). For example, Abdelrahman L.Z. (WO 98/37173) demonstrated the production of fine cells (suspension cells) from sugar cane in liquid cultures, which are capable of producing somatic embryos in a bioreactor. However, the production of sugarcane embryos from thin cells comes up against some difficulties, such as, but not limited to: a more genotype-dependent process; habituation of the cells leading to low conversion of somatic embryos and, consequently, germination; and a high rate of albino seedlings.

In addition, the liquid cultivation of plant cell aggregates, especially embryogenic callus, presents other problems, such as the homogeneous distribution of mass (oxygen and nutrients) throughout the aggregated cell structure. Plant cell aggregates create gradients of soluble components in the culture medium, such as oxygen ($O_2$), due to the physical barrier established by the circumference of the cell aggregate, as well as a gradient of growth factors and other metabolites that influence, among other processes, cell differentiation.

This physical barrier means that the stage of development (differentiation) of the callus is not uniform throughout the cell cluster. One of the only tools available so far to overcome this problem is the use of bioreactors whose growing medium is agitated in order to homogenize the distribution of nutrients and oxygen. This agitation is commonly done by impellers, oxygen *input* hoses and the use of *baffles,* but they all generate damaging shear forces.

Cell culture systems based on rotating containers, for example, are also known in the state of the art. For example, Georgiev et al (2014) describe a rotating drum system consisting of a rolling apparatus and an autoclavable plastic or glass flask placed on top of it. A stainless steel mesh or polyurethane foam mat is placed inside the flask to support the explants. When the device is rotating at low speed, the immobilized plants are periodically immersed and exposed to the ambient air. When growing adventitious or hairy roots, it is not necessary to install an internal support, as the roots are adsorbed to the walls of the flask by adhesion. This system, however, has a higher shear stress due to mechanical mixing and the lack of ventilation and internal atmosphere exchange options.

Other authors (Berson et al 1198, Sun, 1999, Sajc et al. 2000, Alkoaik et al, 2018, Kaya et al. 2018), describe highly complex, costly and high-maintenance rotary culture systems, with oxygen *inlets,* active gas exchange membranes, and systems for injecting and maintaining the culture medium.

To date, there is no simple solution that allows the internal fluid dynamics of cell culture bioreactors to enable an even distribution of oxygen and nutrients to all the cells in the plant cluster without the need for an *impeller* or a substance *inlet* which significantly increase the shear forces in the reactor.

Embryogenic suspension cultures are particularly suitable for large-scale production due to the absence of repetitive handling of plant tissue, the potential to easily scale up the process and the relative ease of automating the process. Developing the process into a practical production system ideally requires that single embryos are produced, that the embryos are at the same stage of development and that there is ultimately a high conversion rate into seedlings.

Furthermore, to date, no method of cultivating sugarcane cells has been described in the state of the art, especially the cultivation of sugarcane aggregates and callus in liquid media using rotary cultivation systems.

There is therefore a clear need to develop a process for cultivating cell aggregates in liquid media, especially for the production of somatic plant embryos, in which the transport of mass (oxygen and nutrients) to the nucleus of the cell aggregates is adequate, but without generating shear stresses that lead to the death or cell disintegration. It is essential that the boundary layer between the liquid medium and the wall of the cell aggregate is somehow crossed without causing further damage to the cells of the aggregate.

Thus, the present invention describes an easy and economically advantageous method for cultivating cell aggregates on a commercial scale, guaranteeing the correct transportation of nutrients and oxygen to their cells without generating hydrodynamic disturbances in the cultivation medium that cause their disintegration and/or death. This system is based on rotating containers, is accessible to automation and mechanization, and makes it possible to modify the boundary layer of cellular aggregates, allowing for better mass transfer with low physical damage from disintegration.

Summary of the invention

In one aspect, the present invention provides a process for cultivating cell aggregates in a system of containers rotating around their own horizontal axes, comprising:

    (i) the inoculation of cell aggregates; and
    (ii) the cultivation of said cell aggregates in said rotating containers;

wherein the coefficient between the angular speed of rotation of the containers and their respective radii is calculated according to the Froude equation (Fr) and varies from at least about 0.01 to 0.2.

In one embodiment, the coefficient between the angular speed of rotation of the containers varies from at least about 0.025 to 0.17, preferably from at least about 0.05 to 0.15.

In a second embodiment, the containers have an inclination with respect to the horizontal surface of 0° to 25°, preferably 0° to 20°, even more preferably 0° to 15°, particularly 15°.

In another embodiment, the angular speed of the containers is at least 10 rpm (1.05 rad/sec) to 45 rpm (4.71 rad/sec), preferably 15 rpm (1.57 rad/sec) to 40 rpm (4.19 rad/sec), more preferably 20 rpm (2.09 rad/sec) to 35 rpm (3.67 rad/sec), even more preferably 25 rpm (2.62 rad/sec) to 30 rpm (3.14 rad/sec).

In a further embodiment, the headspace of the container varies from 50 to 20%, more preferably from 40 to 30%, and particularly the headspace of the container is around 30%.

In another embodiment, cell aggregates are aggregates of plant cells.

In a further embodiment, the plant cell aggregates are sugarcane embryogenic calluses.

In a preferred embodiment, sugar cane is a recalcitrant plant.

In other respects, the present invention provides cell aggregates cultivated by the cell aggregate cultivation process as described above.

In a further aspect, the present invention provides a method for micropropagating plant cells comprising:

    (i) selecting a plant of interest to be multiplied;
    (ii) establishing mature or meristematic explants of said plant in vitro;
    (iii) cultivating the explants in culture media suitable for forming dedifferentiated cell aggregates;
    (iv) inoculating said dedifferentiated cell aggregates into containers and proceeding with their cultivation by means of the cell aggregate cultivation process as described above.

In a first embodiment, the aggregate of plant cells is sugarcane embryogenic callus.

In a preferential embodiment, sugar cane is a recalcitrant plant.

In yet another aspect, the present invention provides a method for maturing somatic embryos, comprising the following steps:

    (a) selecting a plant of interest to be multiplied;

(b) establishing mature or meristematic explants of said plant in vitro;

(c) cultivating the explants in culture media suitable for forming dedifferentiated cell aggregates;

(d) producing somatic embryos from the cultivated cell aggregates of step (c); and

(e) promoting the maturation of the somatic embryos in rotating containers;

where the coefficient between the angular speed of rotation of the containers and their respective radii varies from at least around 0.01 to 0.2 and is calculated according to the Froude equation (Fr).

In one embodiment, the coefficient between the angular speed of rotation of the containers varies from at least about 0.025 to 0.17, preferably from at least about 0.05 to 0.15.

Brief description of the figures

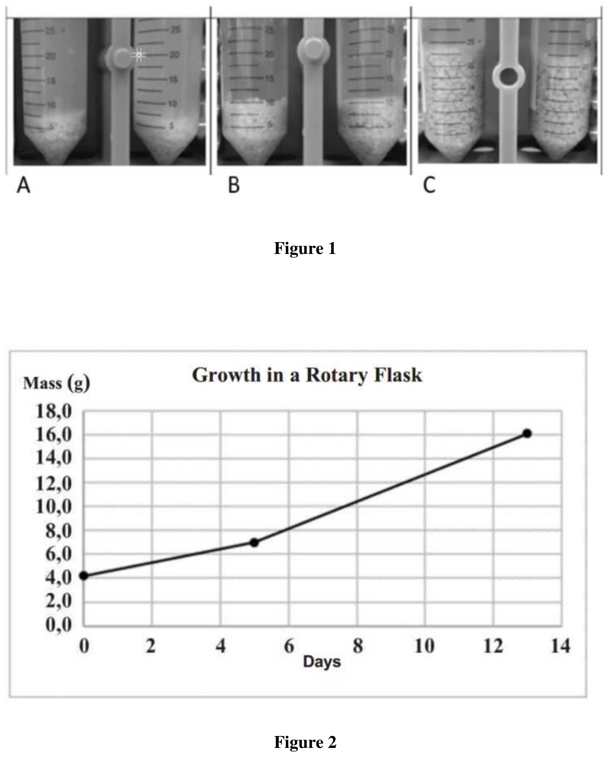

Figure 1: Result of the process of cultivating cell aggregates (callus) of sugar cane in two cylindrical containers after 5 and 13 days of cultivation according to the process of the present application. A: mass of cell aggregates (callus) of sugarcane inoculated in the two containers for cultivation according to the process of the present invention at time zero. B and C represent the mass of cell aggregates (callus) of sugarcane at the end of the cultivation process in 5 (B) and 13 (C) days, respectively. B: represent the total cell mass cultivated for 5 days in the container with a filter in the lid (i) and only with it semi-threaded (ii); C: represent the total cell mass cultivated for 13 days in the container with a filter in the lid (i) and only with it semi-threaded (ii).

Figure 2: Growth graph (in mass (g)) of sugar cane callus cultivated according to the process of the present invention for up to 13 days in the containers.

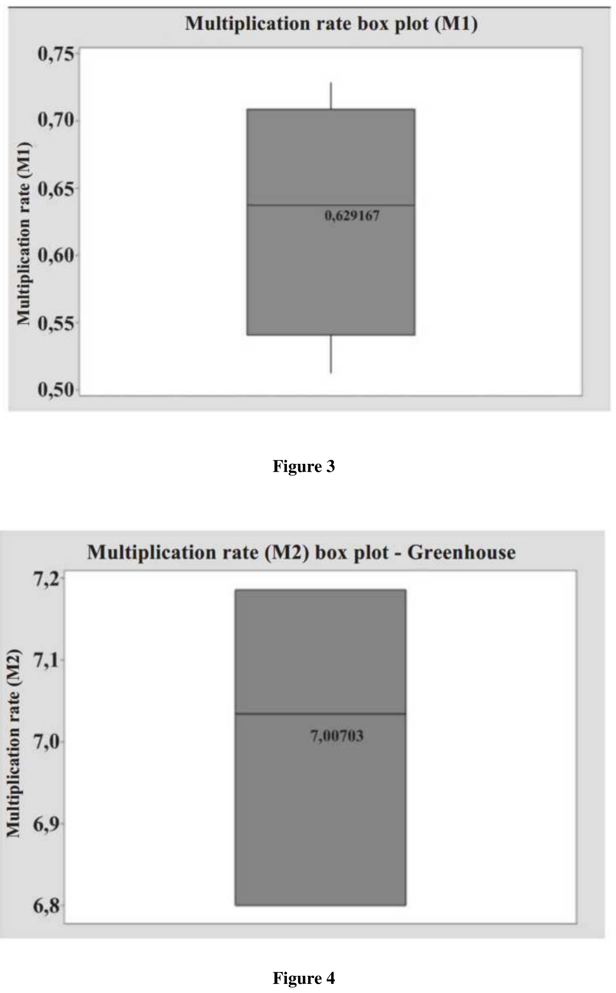

Figure 3: *Boxplot* of the multiplication rate of sugarcane callus at multiplication stage 1 (M1) cultivated according to the process of the present invention. For multiplication 1 (M1), 6g of callus were inoculated into each 200mL of liquid medium and cultivated for a total of 28 days, with 3 treatments of the culture medium, changing 50% of the medium each time. The M1 multiplication rate reached values of over 0.7x before being inoculated in multiplication phase 2 (M2).

Figure 4: *Boxplot* of multiplication rate 2 (M2) of sugarcane callus at multiplication stage 2 (M2) cultivated according to the process of the present invention. For multiplication 2 (M2), 4g of callus with an embryogenic profile were inoculated into each 200mL of culture medium and cultivated for a total of 14 days, with 1 handling of culture medium and a 50% change of medium. The M2 multiplication rate was over 7x.

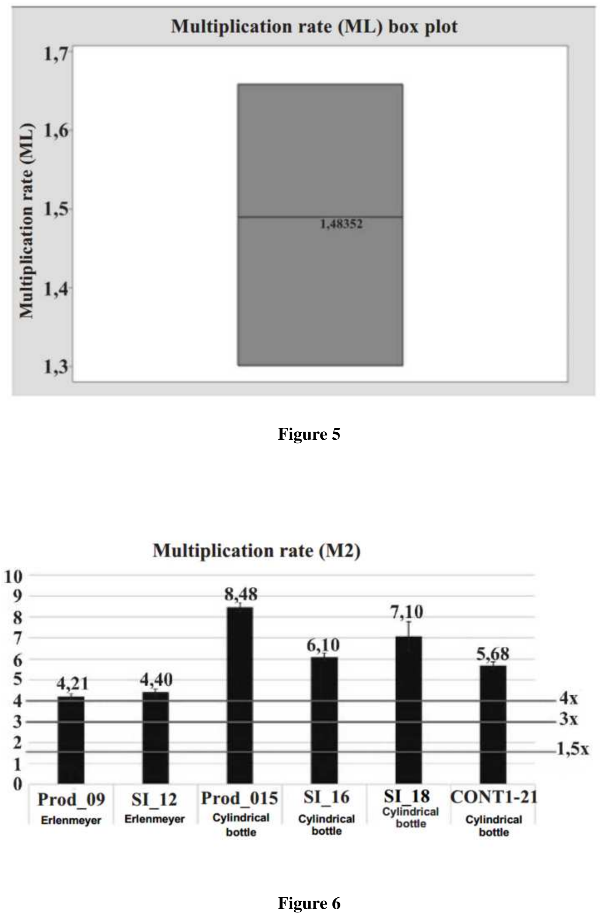

Figure 5: *Boxplot* of net maturation rate (ML) of sugarcane embryogenic callus cultivated according to the process of the present invention. The multiplication rate in the liquid maturation (ML) phase reached values of more than 1.5x.

Figure 6: Comparative graph between multiplication rates 2 (M2) of sugarcane callus using Erlenmeyer flask and the rotating containers of the present invention. The multiplication rate of callus grown in the rotating containers according to the process of the present invention was 50 to 100% higher (approximately 6 to 8x multiplication rate) than the multiplication rate of callus grown in Erlemenayer (approximately 4x multiplication rate).

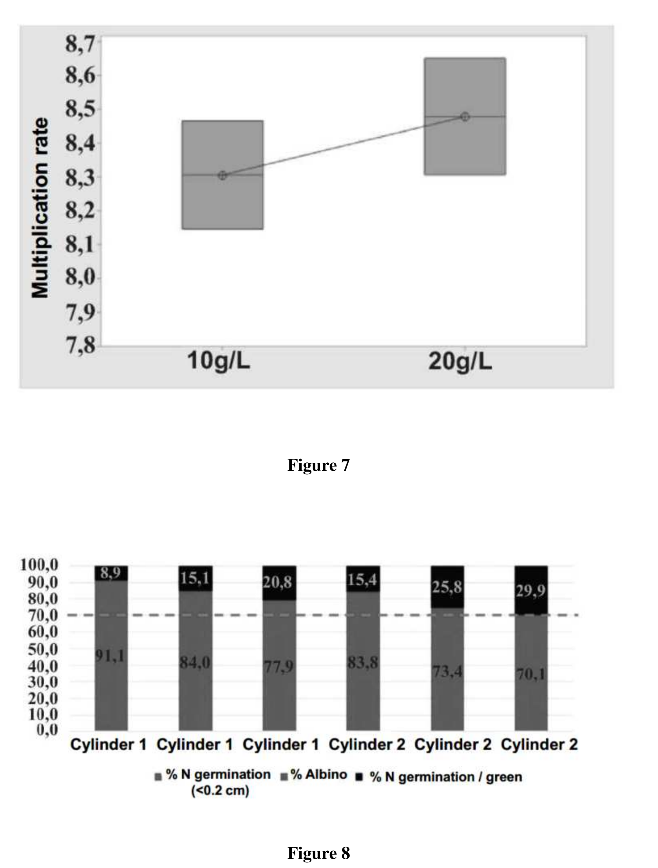

Figure 7: *Boxplot* comparing the amount of sugarcane embryogenic callus produced after multiplication phase 2 (M2) using the rotating containers of the process of the present invention starting from two different inocula concentrations, 10g/L and 20g/L.

Figure 8: Graphs of the average germination percentage index of sugarcane variety embryogenic calluses cultivated for 17 days according to the process of the present invention.

Detailed Description of the Invention

Unless otherwise defined, all technical and scientific terms used herein have the same meaning as understood by a person skilled in the art to which the invention pertains. The terminology used in describing the invention is intended to describe particular realizations only, and is not intended to limit the scope of the teachings. Similarly, unless otherwise indicated, all numbers expressing quantities, percentages and proportions, and other numerical values used in the descriptive report and in the claims must be understood as being modified, in all cases, by the term "about." Thus, unless otherwise stated, the numerical parameters shown in the descriptive report and in the claims are approximations that may vary, depending on the properties to be obtained.

It is a first objective of the invention to provide a method for growing aggregated cell masses in liquid culture medium in containers that allow sufficient nutrition and oxygenation of all the cells in the aggregates. The correct delivery of nutrients and oxygen combined with a cultivation environment without major hydrodynamic disturbances allows for the cultivation of cell aggregates with greater multiplication efficiency, maintenance of biological viability and an undifferentiated stage.

Furthermore, it is a primary objective of the invention to cultivate plant cell aggregates, such as but not limited to callus, in order to enable their high cell multiplication without adverse effects such as oxidation and cell dispersion in the form of thin cells.

Additionally, the first objective of the present invention comprises the cultivation of plant calluses according to the process of the present invention so that their development stage remains undifferentiated, more specifically with an embryogenic profile, due to the correct establishment of a cultivation environment whose distribution of oxygen and nutrients is uniform for all plant callus cells without shear stresses and/or other stimuli that direct their development to the detriment of their embryogenic profile.

Even more specifically, the first objective of the present invention is the multiplication of sugar cane plant callus with an embryogenic profile, allowing a high germination rate to be obtained under suitable conditions.

The first objective of the present invention is achieved by means of a cell aggregate cultivation process based on the inoculation of a portion of cell aggregates in a system of containers that promote the multiplication of cell aggregates and maintain their viability and more undifferentiated state due to the greater homogeneity of the distribution of oxygen, nutrients and other compounds to all the cells of the cell aggregate.

By "inoculum" we mean all biological material that is inoculated at time 0 (T0) (*i.e.*, at the start of cultivation) or at any other time during cultivation. Those skilled in the art understand that for each type of crop, there will be a more suitable type of inoculum. Thus, by way of example, but not limitation, the inocula of the present invention can be eukaryotic cells, eukaryotic cell aggregates, callus, cells from meristematic tissues, apical buds, lateral buds, root buds, immature embryos, zygotic embryos and somatic embryos, among others. Preferably, the inocula of the present invention are plant cells. Even more preferably, the plant cells are sugar cane calluses.

"Cell aggregates" means any set of plant cells that form a unified, cohesive cell mass whose appearance is that of an individualized structure in which its cells are interconnected and grouped together.

By way of example, but not limitation, examples of cell aggregates of the present invention can be formed by plant cells such as callus, meristematic cells and cells arising from apical, axillary and root buds, tumor cell assemblies, immature embryos, zygote plant embryos, somatic embryos, plant tumor cells, among others. Preferably, the cell aggregates of the present invention are plant cell aggregates. Even more preferably, plant cell aggregates are plant calluses. In particular, plant calluses are from monocots, particularly sugar cane. Furthermore, in a particular aspect of the invention, sugar cane plant calluses can be embryogenic plant calluses.

"Cell multiplication" means the process by which a cell divides and/or the process by which the cells of a cell aggregate divide, increasing the cell mass contained in that aggregate and consequently the total cell mass of the culture.

The cell multiplication rate of a cell culture can be measured by several methods, all of which are widely known in the state of the art, such as, but not limited to: measurement of optical density (OD) at the beginning and end of the culture, weighing of dry mass, weighing of total mass, turbidimetry, Neubauer plate count, cell count on a plate, by measuring the elements of the culture medium (substrate, consumption/production of $O_2$, $CO_2$ and other elements, measurement of the rheological properties of the culture medium, among others. Particularly in the case of the present invention, the multiplication rate of cell aggregates can be measured by optical density evaluations and/or by weighing the mass of cells during cultivation.

By "maintenance of viability" we mean the ability of a cell aggregate to remain alive under the imposed culture conditions and for the total duration of the culture. In particular, maintaining the viability of the cell aggregates of the present invention refers to the ability of the cell aggregates cultivated according to the process of the present invention to follow a route of morphogenesis and/or embryogenesis after cultivation, indicating that cultivation has not hindered their further development. Even more particularly, the cell viability of plant cell aggregates cultivated according to the present invention is their ability to differentiate after cultivation. More particularly, the maintenance of cell viability described in the present application is the ability of plant calluses cultivated according to the process of the present invention to form somatic embryos which, in turn, will regenerate into new mature plants.

The cell aggregate cultivation process of the present invention can be defined as a process for cultivating cell aggregates in a system of containers rotating around their own horizontal axes comprising:

(i) the inoculum of cell aggregates; and
(ii) cultivation of cell aggregates in rotating containers.

"Rotating containers" means all containers suitable for use in the process of the present invention. By way of example and not limitation, these containers can be of any size and shape, as long as they do not have abrupt corners that prevent the smooth movement of the culture medium inside and/or its rotation on its own horizontal axis. The containers suitable for carrying out the cell aggregate cultivation process of the present invention can all be those with a shape suitable for rotating on their own horizontal axes.

For example, containers can be *Schott*-type bottles, Fálcon tubes, cylindrical roll-on bottles, cylindrical, conical, rounded or spherical bottles made of plastic or other polymer such as polystyrene, polypropylene or polycarbonate, among others. These include cylindrical, conical, rounded or spherical glass bottles, borosilicate glass bottles, among others, and cylindrical, conical, rounded or spherical metal bottles. The containers may or may not have holes in the lid for gas exchange, have a retention membrane (filter) with sufficient porosity to ensure that the growing medium is not contaminated with various contaminants (biological or otherwise), or have conventional lids half screwed onto the container. Additionally, in the case of containers without threads for fitting conventional lids, the lids can be any material capable of preventing the entry of contaminants and the overflow of the internal culture medium, such as, but not limited to: cotton, gauze, film paper, plastic materials, rubber, corks, plugs, among others. Regarding the way in which the cell aggregate cultivation process is conducted, it is necessary to note that the rotation of the containers on their own horizontal axes moves the internal liquid in order to enable the crossing of the liquid-solid barrier between the culture medium and the cell aggregate, thus distributing nutrients, oxygen, and other compounds to the cells of an inoculated cell aggregate.

In this sense, the present inventors have identified that, in order to obtain adequate movement to allow adequate mass transfer to all the cells of the aggregates while reducing the physical damage of disintegration, it is necessary to check the coefficient between the radius of the container and its angular speed, according to the Froude equation (Fr) below:

$$Fr = \frac{\emptyset\omega^2\ R}{g}$$

$\emptyset\omega$ = angular speed of the container; [$\Omega$ (rad/s)]
R = radius of container; [ Radius (m)]
g = acceleration due to gravity. [ Gravity (m/s$^2$)]
Fr = A dimensionless number

[0056] According to the present invention, the Froude coefficient ranges from at least 0.01 to 0.2, preferably from 0.025 to 0.17, even more preferably from 0.03 to 0.15, allowing the liquid-solid boundary layer of the cell aggregates to be broken and mass transport into the cell aggregate to occur homogeneously and efficiently. In particular, the variation of the Froude Coefficient between around 0.05 and 0.15 means that the cells of plant cell aggregates such as callus maintain a high embryogenic profile and are viable for both transformation and plant propagation.

In addition, the internal movement characteristic of the fluids that is obtained by keeping the Froude coefficient within the ranges described in the present invention allows the aggregates to have their boundary layer constantly altered, solving one of the main restrictions for mass transport phenomena mentioned above.

The rotations that the flasks are subjected to under their own horizontal axes and the sizes of the flasks can vary freely without any limitation, as long as they meet the variation range of the Froude coefficient (Fr) established above.

Preferably, however, the angular speed of the containers is at least 10 rpm (1.05 rad/sec) to 45 rpm (4.71 rad/sec), preferably 15 rpm (1.57 rad/sec) to 40 rpm (4.19 rad/sec), more preferably 20 rpm (2.09 rad/sec) to 35 rpm (3.67 rad/sec), even more preferably 25 rpm (2.62 rad/sec) to 30 rpm (3.14 rad/sec).

The containers can be rotated using any system capable of anchoring the containers in a horizontal position and promoting their rotation under their horizontal axis. By way of example and not limitation, rotation systems can be: magnetic induction rotation systems, air impulse rotation systems, conveyor belt rotation systems, among others.

Furthermore, the containers can have inclinations in relation to the horizontal surface on which they are resting of at least about 0° to 25°, preferably at least about 0° to 20°, even more preferably at least about 0° to 15°, particularly about 15°.

In addition, it is preferable to fill the containers at least 20, 25, 30, 35, 40, 45, 50, 55, 60, 65 and 70% of the time with medium, so that gas exchange between the *headspace* and the surface of the liquid takes place properly. Furthermore, the rate at which containers are filled with growing medium can vary, depending on a number of characteristics, such as, but not limited to, the metabolic needs (nutrients, oxygen and other compounds) of the cell aggregate being grown, the total volume of the container, the type of material that makes up the containers (*i.e.* more or less porous, glass, polymer, plastic, *etc.*), among others. Preferably, but not limitatively, the *headspace* of the container varies from 50 to 20%, more preferably from 40 to 30%, particularly the *headspace* of the container is around 30%.

A first possible application of the first objective of the present invention is to cultivate callus cell aggregates from plant explants, such as meristematic tissue explants, palm hearts or leaf tissues in order to generate somatic embryos via indirect somatic embryogenesis (Ho and Vasil 1983a; Lee 1987; Chowdhury and Vasil 1993; de Alcantara et al. 2014, (Liu, 1993, J. Plant Physiol. 141:714; Brisibe et al., 1994, New Phytol. 126: 301; Blanco et al., 1997, Plant Cell Tiss Organ Cul 51: 153)) and its consequent regeneration in mature plants. In this sense, it should be noted that the process of somatic embryogenesis, which is widely known by those skilled in the art, comprises at least the stages of callus induction, multiplication of embryogenic cultures, maturation and regeneration of embryogenic calluses and/or somatic embryos in mature plants.

"Embryo" or "Mature Embryo" is defined here as the earliest recognizable multicellular stage of an individual, capable of differentiating and forming a complete seedling, containing aerial stem part and aerial root part, i.e. bipolar structure.

"Somatic embryos" as used by the present invention are independent bipolar structures, with a radical apex and a cauline apex, joined by a procambium that will give rise to the vascular tissues.

Commonly, in indirect somatic embryogenesis processes, the induction of *callogenesis* occurs through the phytoregulator 2,4-dichlorophenoxyacetic acid (2,4-D; auxina; WO9001058; Guiderdoni & Demarly, 1988, Plant Cell Tiss. Organ Cult. 90: 71; Ali et al., 2007, Pak. J. Bot. 39: 1961), being used alone and often in combination with other hormonal agents (kinetin, IAI, BAP, GA3). Different concentrations of 2,4D are reported for inducing callogenesis, but it is also commonly used in the other stages of the process, such as in the multiplication, maturation and regeneration of embryogenic cultures (Naz et al., 2008; Jahangir et al., 2010). The increase in the frequency of embryogenic calluses in indirect somatic embryogenesis processes is also observed through the supplementation of callus induction and multiplication media with specific amino acids, such as proline (Gill e tal., 2004; D2, Kaur & Kapoor, 2016; Bianc et al., 2002; George, E. F. et al., 2008; Pien, S. et al. 2001). Different combinations of amino acids have already been evaluated, with no single ideal combination reported for sugar cane crops. In addition, the use of casein hydrolysates is also common, not only as a nitrogen source, but also as a general adjuvant for the growth, induction, maintenance of embryogenicity and regeneration of plants in *in vitro* cultures, and its presence in the culture media is very common (GANDONOU et al, 2005). The addition of activated charcoal at different stages in the process of somatic embryogenesis or organogenesis of plant cells is also widely described, and in some cases is reported to be essential due to the high release of phenolic compounds during the cultivation of some species/varieties. The role of activated charcoal in controlling the effect of phenolic substances released into the culture medium by the explants is often related to increasing the embryogenic potential of these cultures (Kaur & Kapoor, 2016).

However, the process of cultivating cell aggregates in this application, specifically plant calluses with an embryogenic profile, makes it possible to reduce the exposure of tissues to phytoregulators, especially 2.4D, which is known to cause significant somaclonal variations in cultivated tissues. This is because the high multiplication rate provided by the process of the present invention reduces the total cultivation time, and therefore the exposure time to the phytoregulator, as well as creating an oxygen and nutrient distribution environment that favors embryogenicity.

The process according to the present invention can be carried out using any culture medium suitable for cell cultivation.

By way of example and not limitation, the cultivation process of the present invention can use a basal culture medium including the nutrient formulation of Murashige & Skoog (MS) (Murashige & Skoog, 1962, Physiologia Plantarum 15 473) or Gamborg (Gamborg et al., 1968, Exp. Cell Res 50 151), but other culture media suitable for plant culture, as well as their modifications, can also be used.

The medium can also include carbohydrates. Preferably, the carbohydrate is sucrose, preferably at a concentration of 20 g/L to 30 g/L.

Additional components of the medium are selected from the group consisting of antioxidants, vitamins and phytoregulators auxins and/or cytokinins, depending on the genotype. Preferably, the culture medium can additionally include citric acid, at a concentration of 90 mg/L to 210 mg/L, more preferably 100 mg/L to 150 mg/L and/or ascorbic acid, at a concentration of 90 mg/L to 210 mg/L, more preferably 100 to 150 mg/L.

The culture medium can also include 0.5 mg/mL to 1 mg/mL nicotinic acid, 5 mg/mL to 10 mg/mL thiamine-HCl, 0.5 mg/mL to 1 mg/mL pyridoxine-HCl, 1 mg/mL to 2 mg/mL glycine and 50 mg/mL to 100 mg/mL inositol.

Thus, preferably, the composition of the culture medium consists of MS basal medium with pH adjustment to 5.7 $\pm$ 0.1 after adding all the components and phytoremediators.

The medium may additionally include one or more auxins or compounds with auxin-like activity, selected from the group consisting of $\alpha$-naphthaleneacetic acid (NAA), 2,4-dichlorophenoxyacetic acid (2,4-D), 3-amino-2,5-dichlorobenzoic acid (AD), p-chlorophenoxyacetic acid (CPA), 3-indolebutyric acid (IBA), indole-3-acetic acid (IAA), 2,4,5-trichlorophenoxyacetic acid, phenylacetic acid, picloram, $\beta$-naphthoacetic acid, dicamba, and trans-cinnamic acid. Preferably, the auxin is $\alpha$-naphthaleneacetic acid (NAA), 2,4-dichlorophenoxyacetic acid (2,4-D), 3-amino-2,5-dichlorobenzoic acid (AD), p-chlorophenoxyacetic acid (CPA).

The medium can also include one or more cytokinins or compounds with cytokinin-like activity, selected from the group consisting of 6-benzyladenine (BA or BAP), kinetin (KIN), zeatin, $\alpha$-isopentyladenosine and diphenylurea. Preferably, the cytokinin is 6-benzyladenine (BA or BAP) or kinetin (KIN).

"Plant calli" refers to nodular, yellowish, actively dividing tissues that are not organized, but have a high potential for differentiation, formed by dedifferentiated or already differentiated cells that are actively dividing, the origin of which can be naturally caused by injuries to the plant or by induction in a specific culture medium.

Also, "embryogenic plant callus" or "embryogenic callus" means dedifferentiated cells in the multiplication stage, originating from an explant, with the potential to form somatic embryos. Those skilled in the art are familiar with the composition of culture media suitable for generating embryogenic calli. Preferably, the culture media used are based on compositions comprising ingredients such as MS salts (Murashige and Skoog, 1962), sucrose, B5 vitamins and, optionally: selected amino acids from the group comprising proline and asparagine; casein hydrolysate; citric acid; mannitol; copper sulphate; glycine; gelling agent; auxins; antibiotics; acetoseringone; and selection agents. *"Callogenesis",* as used here, refers to the process of inducing the formation of calli from a tissue or fragment thereof with embryogenic potential.

In addition, the process of cultivating cell aggregates of the present invention not only enables greater production of embryogenic calli, but can also reduce somaclonal variations arising from the use of phyto-regulators and/or other compounds during the multiplication and maturation stages of somatic embryos, as already mentioned.

In view of the above, the present invention contributes to the cost-effective production of plant material such as embryogenic calli, somatic embryos and seedlings on a commercial scale.

A third possible application of the first objective of the present invention comprises the production of plant calli, with a more or less embryogenic profile, from plant explants, for plant transformation via infection by *Agrobacterium spp,* mutagenesis, genome editing, genetic modification through genome editing, performance of *screening* tests of new *traits,* production of synthetic seeds, among others.

A fifth possible application of the first objective of the present invention comprises the multiplication and maintenance of the dedifferentiated profile of plant cell calli arising from cell explants for *in vitro* plant micropropagation and/or genetic transformation (as mentioned above) and/or for storage.

"Storage" means both cryopreservation and encapsulation processes. "Cryopreservation" refers to the preservation of biological material under ultra-low temperature conditions in liquid nitrogen at -196°C, or in its vapor phase, at (-150°C). "Encapsulation" means any process of storing calli in any type of structure that contains them.

These achievements of the present invention make it possible to produce, for example, a large quantity of embryogenic plant tissues, especially callus cell aggregates, synchronously. In addition, these achievements make it possible to obtain high rates of cell multiplication as a result of the optimum conditions for transporting mass (nutrients and oxygen) to the cell calli without generating harmful shear forces. In addition, the cell aggregates produced by the process of the present application have a high germination rate and little oxidation.

By way of example and not as a limitation, the cultivation process of the present invention makes it possible to produce more sugarcane plants via indirect somatic embryogenesis compared to other vegetative propagation methods commonly used and widely known by those skilled in the art.

Both the growth and multiplication of embryogenic calli and the subsequent development of embryos to maturity for regeneration can be carried out in liquid media according to the process of the present invention. This makes it possible to handle a large number of propagules with less need for handling personnel and consequently lower contamination rates of the media due to the low need for handling them. Furthermore, the product of indirect somatic embryogenesis is an embryo capable of developing into a regenerated plant with very little additional work.

It is a second objective of the present invention to provide cell aggregates whose cultivation and production originates from the process of cultivating cell aggregates by means of containers that allow the homogeneous nutrition and oxygenation of the cells of the aggregates.

Specifically, it is a second objective of the present invention to provide cell aggregates in the form of plant calli at early stages of differentiation, more precisely with a marked embryogenic profile and low oxidation.

More specifically, the second objective of the present invention is to provide sugarcane plant calli with a marked embryogenic profile and low oxidation, thus leading to high germination rates of this material when exposed to appropriate conditions. Alternatively, the embryogenic calli provided according to the cultivation process of the present invention have an embryogenic profile and can be used for genetic manipulation by means of particle bombardment, indirect transformation via infection with specific bacteria, genomic editing, mutagenesis and other methods widely known to those skilled in the art. In addition, calli with an embryogenic profile produced by the process of the present invention can be stored.

The second objective of the present invention is achieved by supplying cell aggregates from any organism, as long as they are provided by the rotating container cultivation system of the present invention.

A third objective is to provide a method for micropropagating plant cells, comprising the selection of plant explants, their establishment in suitable culture media to induce multiplication, isolation and inoculation of the cell aggregates generated in rotating containers to carry out the process of growing the cell aggregates as described in the first objective of the present invention.

In addition, the third objective of the present invention is to micropropagate plant cells by inducing *callogenesis* in the plant explants and then inoculating them into the rotating containers of the present invention for multiplication according to the cell cluster cultivation process of the present invention. The calli generated from the explants are multiplied while maintaining their undifferentiated profile so that they can later be inoculated into specific media to develop new plants.

In addition, the third objective of the present invention is to enable greater *in vitro* vegetative production of sugarcane plants by means of the cultivation and large-scale multiplication of cell aggregates derived from the plant's explants.

The third object of the invention comprises a highly efficient method for generating genetically identical plants from a cell or explant of a plant of interest.

The method of micropropagating plant cells, according to the third object of the invention, comprises:

(a) select a plant of interest to be multiplied;
(b) establish mature or meristematic explants of the plant *in vitro;*
(c) cultivate the explants in culture media suitable for the formation of dedifferentiated cell aggregates;
(d) to inoculate these dedifferentiated cell aggregates into the containers with rotation under their horizontal axis as defined above in the first object of the present invention and to proceed with their cultivation by means of the process, also defined above in the first object of the present invention.

This method first involves selecting the plant of interest, which can be selected from any of the groups consisting of: angiosperms, gymnosperms, bryophytes and pteridophytes. Preferably monocots or dicots. Even more preferably monocots of the *Poacea* class. Preferably, the plant of interest is a monocot. Of even greater interest, the plant selected is sugarcane (*Saccharum spp.*), which can be natural sugarcane plants, improved varieties, genetically modified sugarcane plants (by genomic editing or conventional transgenics) or any of these options involving recalcitrant genotypes. Even more preferentially, the explant is isolated from recalcitrant sugarcane plants, both for plant transformation and *in vitro* cultivation, which are widely known to those skilled in the art.

The second stage of the plant cell micropropagation method involves establishing explants or isolating plant cells/tissues capable of being cultivated *in vitro.* Those skilled in the art are well aware of the methods for isolating plant explants. More preferably, the explants are mature explants or from meristematic regions of the plants of interest.

"Explants" are fragments of living plant tissue with the potential to dedifferentiate and induce new cells, provided they are given the right stimuli. Explants can be, but are not limited to, a leaf explant, stem or stalk explant, meristematic explant, root explant, reproductive structure explant, seed explant, bud explant, shoot explant, cell explant, protoplast explant (cell without cell wall), zygote embryo explant, anther explant, among others. Cell explants, protoplast explants (cells without a cell wall), zygotic embryo explants, anther explants, among others. Examples of explants: appropriate tissues obtained from transgenic or non-transgenic plants, including tolete or palm heart (set of young, curled leaves containing the apical meristem), leaf blade, axillary buds, stems, stem apex, leaf sheath, internodes, petioles, floral stems, root or inflorescence, rhizome. A relevant biological property is that the appropriate tissues to be used must contain dividing cells with the potential for growth and differentiation.

The third stage of the plant cell micropropagation method involves growing these explants in liquid or solid culture media containing the necessary nutrients and cell proliferation inducers to form cell aggregates from the explants. These cell aggregates formed from the explants may be embryogenic plant calli stimulated by hormones, carbon sources, among others.

Finally, the fourth and last stage of the plant cell micropropagation method of the present invention comprises the inoculation of said cell aggregates, more preferably cell aggregates with a dedifferentiated profile, in the containers with rotation around their own horizontal axes of the present invention.

The inoculum of the cell aggregates can be around 5 to 45g/L, more preferably 10 to 20 g/L or most preferably 10 or 20 g/L.

Finally, a fourth objective of the present invention is to provide a method for maturing somatic embryos and cell aggregates in liquid medium generated in rotating containers to carry out the process of cultivating cell aggregates as described in the first objective of the present invention.

The fourth and final objective of the present invention is to provide a method for maturing somatic embryos comprising the following stages:

a) select a plant of interest to be multiplied;
b) establish mature or meristematic explants of the plant in vitro;
c) cultivate the explants in culture media suitable for the formation of dedifferentiated cell aggregates;
d) inoculate such dedifferentiated cell aggregates into rotating containers and cultivate them according to the method for cultivating aggregated cell masses of the first object of the present invention
e) produce somatic embryos from the cultured cell aggregates from step (d); and
f) promote the maturation of somatic embryos.

The maturation of somatic embryos is understood to be the phase in which the development of somatic embryos is stimulated. This stimulation of the development of somatic embryos to late stages of development can occur in a number of ways, all of which are widely known to those skilled in the art. The strategies commonly employed consist of interrupting the repetitive cycles of cell division and providing the physiological, biochemical and environmental stimuli for cell differentiation, thus giving rise to a large number of mature somatic embryos, of high quality and capable of becoming plants (GUERRA et al., 1999). The main ripening-promoting substances are abscisic acid (ABA), osmotic agents such as polyethylene glycol (PEG), carbohydrates, hexitois and activated charcoal (LAKSHMANAN, 2005). Preferably, the maturation of the somatic embryos produced by the cell aggregate cultivation process of the present invention can be carried out in the rotating containers of the present invention, but with the appropriate culture medium.

Furthermore, the more synchronized the development stages of the cells that make up the cell aggregates (embryogenic calli), the more homogeneous and better the maturation phase of the somatic embryos will be.

Finally, the high quality multiplication of cell aggregates (i.e. subsequent germination) together with the possibility of scaling up the use of rotary bioreactors and their reduced cost makes it possible to scale up this process, meeting the demands for this type of plant material and at substantially lower costs compared to conventional bioreactors.

Examples

Example 1. Growth of cell aggregates in 1L rotating flasks

This example demonstrates the high efficiency of cultivating and multiplying sugarcane embryogenic plant calli in rotating containers with a total volume of 1L.

For this experiment, two cylindrical flasks (*Schott* type) with a *headspace* of approximately 70% were used, one containing a lid with a 0.22 μm filter and the other without a filter and a semi-threaded lid. Both fractures were subjected to rotation about the horizontal axis of up to about 25 rpm.

In addition, the flasks were kept at an inclination of around 15° to the horizontal plane and a mass of plant cell aggregates was inoculated into them, enough for the growing medium to reach an initial concentration of around 14 g/L. This inoculum comes from the culture of sugarcane explants in callogenesis-inducing media.

Figures 1 and 2 show, respectively, that both configurations (filter and non-filter lids) under the conditions mentioned above showed high multiplication of plant calli without their disintegration (Figure 1) at 5 days and 13 days of cultivation.

Figure 2 shows that there was a 4x cell multiplication in relation to the initial mass of cell aggregates in 13 days of cultivation, i.e. at the end of the experiment, a total mass of cell aggregates of approximately 16 g was observed compared to the 4 g of cell aggregates inoculated at the start of cultivation (Time zero).

These results demonstrate the efficiency of multiplication of sugarcane cell aggregates in a working volume of only 300 mL under high rotation (approximately 25 rpm - Fr = 0.034 - 100 mm flask - 25 RPM) and without the disintegration of the aggregates and consequent death of the poorly differentiated cells, as it is possible to observe the slightly oxidized profile of the calli (figure 1).

In addition, it has been shown that both the filter and non-filter caps are efficient for the purpose of the present invention, with no contamination of either medium and no significant difference in the rate of calli multiplication.

Example 2. Growth of cell aggregates in 10L rotating flasks

This example demonstrates the high efficiency of cultivating and multiplying sugarcane embryogenic plant calli in containers with a total volume of 10L.

For this experiment, three cylindrical flasks (FR01, FR02 and FR03) of the *Schott* type with a *headspace* of up to about 70% were used, all containing a semi-cocked lid without a filter, and rotating about 25 rpm with respect to the horizontal axis. (Fr = 0.104 - 300mm flask, 25 RPM

In addition, the flasks were kept at a maximum inclination of around 15° in relation to the horizontal plane and a mass of plant cell aggregates was inoculated sufficient for the culture medium to reach an initial concentration of up to around 10 g/L, as is commonly used in *stirred tank reactor* (STR) cultures. This inoculum comes from the *culture* of sugarcane explants in callogenesis-inducing media.

After 20 days of cultivation, the results of cell aggregate multiplication were as described in Table 1.

TABLE 1 - Data on the cell multiplication rate of cell aggregates cultivated according to the process comprising rotating containers of the present invention. The flasks used have a total volume of 10L with a working volume of 3L (*headspace* of 70%) without filters in the lids and under constant agitation of 25 rpm. An initial inoculum of 25 g of cell aggregates was used (initial cell concentration of approximately 10 g/L). FR: bottle.

| Multiplication rate of cell aggregates | | | |
|---|---|---|---|
| Experiment | Initial mass (g) | Final mass (g) | Multiplication Rate |
| FR01 | 25 g | 197.5g | 7.9x |

(continued)

| Multiplication rate of cell aggregates | | | |
|---|---|---|---|
| Experiment | Initial mass (g) | Final mass (g) | Multiplication Rate |
| FR02 | 25 g | 172.9g | 6.9x |
| FR03 | 25 g | 202.5g | 8.1x |

Surprisingly, it was observed that the multiplication and later germination performance from growing cell aggregates using the 5 and 10 L rotating containers of the present invention (Examples 1 and 2) was equivalent to, and at times superior to, the multiplication/germination performance using bubble-free (STR) bioreactors.

In addition, the rotating vessels in examples 1 and 2 obtained a higher Oxygen Transfer Rate (Kla) than the 7.5L (total volume) bubble-free bioreactor (STR - Bubble Free) with a working volume of 5L under 30 rpm agitation, as shown in Table 2.

For gas-liquid systems, the most common characterization is oxygen transport by the parameter kLa, in which this value represents the oxygen transfer coefficient (CURTIS; TUERK, 2008).

$$OTR = k_La (DO * - DO) (1)$$

In which,

OTR: Oxygen transfer;
KLa: Oxygen transfer coefficient and

(a) represents the interfacial area of the object;

DO: Oxygen concentration in the nucleus of the system;
DO*: Saturation concentration of dissolved oxygen at equilibrium in the medium.

The dynamic method proposed by Chisti (1989) was used to determine (KLa). The dynamic method consists of using an electrode to measure the concentration of dissolved oxygen. By applying a flow of air to the system, saturation occurs, and the electrode is calibrated to 100%. Nitrogen is then applied until the oxygen value reaches zero, calibrating the electrode as zero oxygen.

TABLE 2 - Comparison of the Oxygen Transfer Coefficient (Kla) between the conventional stirred bed bioreactor without bubbles (STR) and the rotating vessels of the present invention with different working volumes.

| Bioreactor Type | Total volume (L) | Working volume (L) | Rotation (rpm) | Oxygen transfer coefficient (Kla) |
|---|---|---|---|---|
| Bubble Free Stirred Bed Bioreactor (STR) | 7.5 L | 5 L | 30 rpm | 1 |
| Rotary flask of the present invention | 5 L | 3 L | 25 rpm | 5 |
| Rotary flask of the present invention | 10L | 5 L | 25 rpm | 1 |

These results demonstrate the high oxygen transfer rate obtained by the rotating containers of the present invention compared to the complex and costly system of bubble-free stirred bed bioreactors (STR ). (Fr = 0.08 5L flask - diameter 240mm Fr = 0.10 10L flask - diameter 300mm).

In addition, the high cell multiplication (7.5x of the initial inoculum) and the visual quality of the callus tissue produced by the rotating containers of the present invention indicate high cell viability with little or no damage caused by the agitation of the containers. Unlike what happens in conventional bioreactors, there is no significant damage to the sugarcane calli cultivated in the containers of the present invention, while the impellers and oxygen inlets of bioreactors commonly produce the disintegration of the callus and/or lesions in it that lead to oxidation and death.

Example 3. Establishment of a Multiplication and Liquid Maturation process to obtain embryogenic calli of the sugarcane variety

In this example, a recalcitrant sugar cane variety from a greenhouse was used. The trial began with 20 palm hearts (R0), which underwent a gradual drying process prior to collection. Tissue swelling was then induced in these explants and 15 leaf discs were inoculated per plate. After 14 days (R1), these explants were moved to multiplication phases 1 and 2 (M1 and M2, respectively) at the following densities:

- Multiplication 1 (M1): 6 g/200 ml (28 days; 3 treatments of 50%);
- Multiplication 2 (M2): 4 g/200 ml (14 days; 1 handling of 50%).

At around 14 days in Multiplication 1, the beginning of embryogenic calli formation was observed, and at 25 days, yellowish/nodular embryogenic calli was observed, which was looser in the liquid medium.

After multiplication, liquid maturation was carried out using a culture medium similar to that used in the previous phase, replacing 2.4-D with 0.1 to 0.5 mg/L abscisic acid and 0.5 to 1 g/L activated charcoal for 4 days. Desiccation took place in a laminar flow (rapid desiccation) until the cell aggregates reached around 40% moisture. The aggregates were germinated using the appropriate culture medium.

The liquid multiplication and maturation stages were carried out according to the process described in the present invention, i.e. with rotating containers. Four rotating containers were used in M1 and three rotating containers in M2, both of 2.5L (total volume).

The average multiplication rates of embryogenic calli in multiplication phases 1 and 2 were, surprisingly, 0.6x and 7x, respectively. Details of these phases can be found in Tables 3 and 4 and Figures 3 and 4, respectively.

TABLE 3 - Table of descriptive statistics for M1.

| Variable | N | N* | Average | Average EP | Standard Deviation | Minimum | Q1 | Median | Q3 | Maximum |
|---|---|---|---|---|---|---|---|---|---|---|
| Multiplication rate 1 | 4 | 0 | 0.6292 | 0.0444 | 0.0889 | 0.5133 | 0.5413 | 0.6375 | 0.7087 | 0.7283 |

TABLE 4 - Table of descriptive statistics for M2.

| Variable | N | N* | Average | Average EP | Standard Deviation | Minimum | Q1 | Median | Q3 | Maximum |
|---|---|---|---|---|---|---|---|---|---|---|
| Multiplication rate 2 | 3 | 0 | 7.007 | 0.112 | 0.194 | 6.800 | 6.800 | 7.034 | 7.186 | 7.186 |

On the other hand, the average multiplication rate of embryogenic calli in the liquid maturation phase was, surprisingly, 1.5x. The details of this phase are shown in Table 5 and Figure 5, respectively.

TABLE 5 - Table of descriptive statistics for net maturity (ML).

| Variable | N | N* | Average | Average EP | Standard Deviation | Minimum | Q1 | Median | Q3 | Maximum |
|---|---|---|---|---|---|---|---|---|---|---|
| Net Maturation Rate (ML) | 3 | 0 | 1.484 | 0.103 | 0.178 | 1.302 | 1.302 | 1.490 | 1.659 | 1.659 |

These data show that the use of the rotating containers of the present invention led to high multiplication and maturation rates of plant cell aggregates (average multiplication rate of around 4x), enabling high production with high quality aggregate material (*i.e*, non-oxidized, uniform and non-segregated aggregates with a highly embryogenic profile).

Based on the surprising data above, the potential for using rotating containers in the multiplication and maturation phases became clear. As a result, some adaptations were made in order to further improve the multiplication rates of the aggregates in the multiplication phases: use of different densities in M1 and M2; more careful disaggregation and selection of calli at the end of M1; and removal of viscosity from the medium through handling in the liquid multiplication phase. With these adaptations, an average multiplication rate of 6.83x was achieved in 14 days in M2, substantially higher than the multiplication rate obtained by multiplying the aggregates in Erlenmeyer flask (approximately only 4x) (Figure 6).

Example 4. Multiplication, Liquid and Solid Maturation and Germination of embryogenic calli of sugarcane varieties grown and produced in rotating containers

Plant material and methodology

The embryogenic calli multiplication trial for the sugarcane variety was started by inducing around 30 palms (R0). Then, in the multiplication phase 1 (M1), an initial density of 6g/200ml of calli was used in the culture medium, which was kept for 25 days with weekly treatments corresponding to a 50% change in the medium.

After phase M1 was completed, the material was cleaned by sieving the calli and washing it (water) under vacuum, followed by multiplication stage 2 (M2), in which two densities were tested (10g/L and 20g/L). The M2 stage lasted 14 days, with 50% handling carried out at 7 days of cultivation.

The material was then sieved and washed (water) and transferred to liquid maturation medium (ML) containing charcoal and abscisic acid for 4 days. The density used in liquid maturation was 40g/L. At the end of liquid maturation, the embryogenic cell aggregates were also sieved and washed (water), then transferred to solid maturation (MS) for 10 days and desiccated.

The embryogenic cell aggregates obtained were germinated in the appropriate culture medium.

Multiplication Phases 1 and 2 (M1 and M2)

Around 14 days into multiplication 1 (M1), embryogenic calli formation began, and at 25 days, yellowish/nodular embryogenic calli was observed, which was looser in the liquid medium. The average rate of formation of selected embryogenic calli at the end of multiplication 1 (M1) was 1.55 x (drained fresh weight) (Table 6).

Table 6 - Multiplication rate of cell aggregates and embryogenic calli formation in M1 (multiplication 1)

| # Container | *input* in M1 (6g/200mL) | Total yield in M1 (g) | Embryogenic calli yield in M1 (g) | Multiplication rate |
|---|---|---|---|---|
| 1 | 6.04 | 32.25 | 10.94 | 1.81x |
| 2 | 6 | 29.2 | 7.94 | 1.32x |

In addition, densities of 10g/L or 20g/L were evaluated as inoculum for the start of multiplication 2 (M2). After 14 days in multiplication 2 (M2), the average growth rate was 8.3x in both inoculation densities (Figure 7), indicating that there was no statistically significant difference in the multiplication rate of cell aggregates in relation to the initial concentration inoculated in the rotating containers for multiplication 2 (M2).

Liquid Maturation Phase

After multiplication phase 2 (M2), the cell aggregates were transferred to the rotating container containing the appropriate culture medium. At this stage, the impact of the inoculation density in M2 on the appearance and quality of the embryogenic cell aggregates at the end of the liquid maturation phase was clear, in which much more embryogenic aggregates were observed from the higher density (20g/L) of M2 to the detriment of mass formation, and few embryogenic aggregates from the material from the lower density (10g/L) in M2.

Germination Stage

Germination data of the variety after 27 days in a light room in semi-solid medium showed the impact of the initial density of the multiplication phase on the quality and germination of cell aggregates.

As expected, germination (greens above 0.5 cm) was observed for only 20% of the 10g/L source material in M2. In addition to the low germinability, high formation of oxidized and non-germinated aggregates was observed, both amounting to around 70%.

On the other hand, vigorous seedlings with germination above 70% were observed in material from M2 20g/L (Figure 8).

Thus, the variable density (20g/L) in Multiplication 2 (M2) represented an increase of around 4x in the germination rate. In addition, a low rate of albinos (<0.6%) was observed for this variety under different germination conditions.

It is clear that the use of rotating containers, especially in the Liquid Multiplication phase, considerably increases the multiplication of plant cell aggregates and also increases their germination rate by around 4x. In other words, the process of the present invention not only enables greater efficiency in the production of cell aggregates of interest, in this case embryogenic calli used for various purposes in plant biotechnology, but also makes them more vigorous during germination.

The examples described represent preferred scopes and it should be understood that the scope of the present invention contemplates other possible variations and is limited only by the content of the claims, including possible equivalents.

**Claims**

1. Cellular aggregate cultivation process, in a system of containers rotating around their own horizontal axes, **characterized by** comprising:

    (i) the inoculum of cell aggregates; and
    (ii) the cultivation of said cell aggregates in said rotating containers;

    where the coefficient between the angular speed of rotation of the containers and their respective radii is calculated according to the Froude equation (Fr) and varies from at least around 0.01 to 0.2.

2. Process, according to claim 1, **characterized in that** the coefficient between the angular speed of rotation of the containers varies from at least about 0.025 to 0.17, preferably from at least about 0.05 to 0.15.

3. Process, according to claim 1 or 2, **characterized in that** the containers have an inclination with respect to the horizontal surface of between 0° and 25°, preferably between 0° and 20°, even more preferably between 0° and 15°, particularly 15°.

4. Process, according to any one of claims 1 to 3, **characterized in that** the angular speed of the containers is at least 10 rpm (1.05 rad/sec) to 45 rpm (4.71 rad/sec), preferably 15 rpm (1.57 rad/sec) to 40 rpm (4.19 rad/sec), more preferably 20 rpm (2.62 rad/sec) to 30 rpm (3.14 rad/sec).

5. Process, according to any one of claims 1 to 4, **characterized in that** the *headspace* of the container varies from 50 to 20%, more preferably from 40 to 30%, and particularly the *headspace* of the container is about 30%.

6. Process, according to any one of claims 1 to 5, **characterized in that** the cell aggregates are aggregates of plant cells.

7. Process, according to claim 6, **characterized in that** the plant cell aggregates are sugarcane embryogenic calli.

8. Process, according to claim 7, **characterized in that** sugar cane is a recalcitrant plant.

9. Cellular aggregates, **characterized in that** they are grown by the process as defined in any one of claims 1 to 8.

10. Method of micropropagation of plant cells, **characterized by** comprising:

    (i) select a plant of interest to be multiplied;
    (ii) establish mature or meristematic explants of the plant *in vitro;*
    (iii) cultivate the explants in culture media suitable for the formation of dedifferentiated cell aggregates;
    (iv) inoculate said dedifferentiated cell aggregates into containers and proceed with their cultivation by means of the process as defined in any of claims 1 to 8;

11. Method, according to claim 10, **characterized in that** the aggregate of plant cells is sugarcane embryogenic calli.

12. Method, according to claim 11, **characterized in that** sugar cane is a recalcitrant plant.

13. Somatic embryo maturation method, **characterized by** the following stages:

    (a) select a plant of interest to be multiplied;
    (b) establish mature or meristematic explants of the plant *in vitro*;
    (c) cultivate the explants in culture media suitable for the formation of dedifferentiated cell aggregates;
    (d) produce somatic embryos from the cultured cell aggregates from step (c); and
    (e) promote the maturation of somatic embryos in rotating containers;

    where the coefficient between the angular speed of rotation of the containers and their respective radii varies from at least around 0.01 to 0.2 and is calculated according to the Froude equation (Fr).

14. Method, according to claim 13, **characterized in that** the coefficient between the angular speed of rotation of the containers varies from at least about 0.025 to 0.17, preferably from at least about 0.05 to 0.15.

**Figure 1**

**Figure 2**

**Figure 3**

**Figure 4**

Figure 5

Figure 6

**Figure 7**

**Figure 8**

## INTERNATIONAL SEARCH REPORT

| International application No. |
| --- |
| **PCT/BR2024/050119** |

**A. CLASSIFICATION OF SUBJECT MATTER**

A01H 4/00(2006.01)i; *A01H 6/46*(2018.01)i; *A01G 22/55*(2018.01)i; C12M 3/02(2006.01)i

According to International Patent Classification (IPC) or to both national classification and IPC

**B. FIELDS SEARCHED**

Minimum documentation searched (classification system followed by classification symbols)

A01H 4/00; A01H 6/46; A01G 22/55; C12M 3/02

Documentation searched other than minimum documentation to the extent that such documents are included in the fields searched

Base de Patentes do INPI-BR; Plataforma Lattes e Google Schoolar

Electronic data base consulted during the international search (name of data base and, where practicable, search terms used)

Espacenet, STN Next (Agricola e Biosis), DWPI

**C. DOCUMENTS CONSIDERED TO BE RELEVANT**

| Category* | Citation of document, with indication, where appropriate, of the relevant passages | Relevant to claim No. |
| --- | --- | --- |
| X | Thorat et al. "Plant regeneration from cell suspension culture in Saccharum officinarum L. and ascertaining of genetic fidelity through RAPD and ISSR markers." 3 Biotech 7, 16 (2017). https://doi.org/10.1007/s13205-016-0579-3 (pages 2-5/12, table 1) | 9 |
| X<br>Y | Akita & Ohta "A simple method for mass propagation of potato (Solanum tuberosum L.) using a bioreactor without forced aeration" Plant Cell Reports 18: 284-287. (1998). DOI: 10.1007/s002990050572 (table 1, page 287) | 1-6, 10, 13-14<br>7, 8, 11, 12 |
| X<br>Y | Akita & Ohta "Development of a system for mass propagation of Colocasia esculenta in large scale without forced aeration". Acta Hort. 440 (1996). DOI: 10.17660/ActaHortic.1996.440.97 (page 555) | 1-6, 10, 13-14<br>7, 8, 11, 12 |

☑ Further documents are listed in the continuation of Box C. ☐ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **14 June 2024** | **25 June 2024** |

| Name and mailing address of the ISA/BR | Authorized officer |
| --- | --- |
| **National Institute of Industrial Property (Brazil)**<br>**Rua Mayrink Veiga, 9, 6° andar, CEP 20.090-910 Rio de Janeiro – RJ**<br>**Brazil** | **Erika TARRE BORGES ANTONELLI** |
| Telephone No. (55 21) 3037-3742, 3037-3984 | Telephone No. +55 21 3037 4528 - 3037 3319 |

Form PCT/ISA/210 (second sheet) (July 2022)

## INTERNATIONAL SEARCH REPORT

| International application No. |
| --- |
| **PCT/BR2024/050119** |

**C.    DOCUMENTS CONSIDERED TO BE RELEVANT**

| Category* | Citation of document, with indication, where appropriate, of the relevant passages | Relevant to claim No. |
| --- | --- | --- |
| X<br>Y | Akita & Ohta "A simple bioreactor system for production of storage organs of chinese yam (Dioscorea opposita Thumb.)" Plant Biotchnology, 19 (5): 353-356. (2002). DOI:10.5511/plantbiotechnology.19.353 (page 354 and figure 1) | 1-6, 10, 13-14<br>7, 8, 11, 12 |
| Y | Orozco-Ortiz et al. "BIT bioreactor increases in vitro multiplication of quality shoots in sugarcane (Sacharum spp. Variety LAICA 04-809)" Plant Cell, Tissue and Organ Culture (PCTOC), 152: 115-128. (2022). DOI: https://doi.org/10.1007/s11240-022-02392-4 (pages 116, 118, 119) | 7, 8, 11, 12 |

Form PCT/ISA/210 (second sheet) (July 2022)

**REFERENCES CITED IN THE DESCRIPTION**

**Patent documents cited in the description**

- US 5587312 A, Holst
- US 5413929 A, Keiichirou and Noburu,
- WO 9837173 A, Abdelrahman L.Z.
- WO 9001058 A

**Non-patent literature cited in the description**

- **ESPINOSA-LEAL et al.** *Planta*, 2018, vol. 248, 1-18
- **FRANKLIN et al.** *Plant Growth Regul*, 2006, vol. 50, 111
- **NAZ et al.** *Sarhad J. Agric.*, 2008, vol. 24 (4), 593
- **BURNER** ; **GRISHAM**. *Crop Sci.*, 1995, vol. 35, 875
- **LIU**. *J. Plant Physiol.*, 1993, vol. 141, 714
- **BRISIBE et al.** *New Phytol.*, 1994, vol. 126, 301
- **BLANCO et al.** *Plant Cell Tiss Organ Cul*, 1997, vol. 51, 153
- **VASIL**. *J. Plant Physiol.*, 1987, vol. 128, 192
- **MANICKAVASAGAN** ; **GANAPATHI**. *Ind. J. Exp. Bio.*, 1998, vol. 36, 832
- **ALI et al.** *Pak. J. Bot.*, 2007, vol. 39 (6), 1961
- **RUI** ; **SEPPO**. *Agri and Food Sci.*, 2004, vol. 13 (4), 363
- **HIMANSHU et al.** *Ind. J. of Agr. Sci*, 2000, vol. 70 (3), 181
- **CHENGALRAYAN** ; **MEAGHER**. *In Vitro Cell & Devel. Biol. Plant*, 2001, vol. 37 (4), 434
- **LAKSHMANAN et al.** *Plant Cell Rep*, 2006, vol. 25 (10), 1007
- **GUIDERDONI** ; **DEMARLY**. *Plant Cell Tiss. Organ Cult.*, 1988, vol. 90, 71
- **ALI et al.** *Pak. J. Bot.*, 2007, vol. 39, 1961
- **MURASHIGE** ; **SKOOG**. *Physiologia Plantarum*, 1962, vol. 15, 473
- **GAMBORG et al.** *Exp. Cell Res*, 1968, vol. 50, 151